# EUROPEAN PATENT APPLICATION

(11) **EP 4 425 174 A1**
(43) Date of publication of application: **04.09.2024**
(21) Application number: 22885313.1
(22) Date of filing: 12.08.2022
(51) Int. Cl.: G01N 33/00, G01N 1/38

(54) **CARBON EMISSION MONITORING SYSTEM**

(30) Priority: 29.10.2021 CN 202111271648
(71) Applicant: Guangdong Brunp Recycling Technology Co., Ltd, Guangdong 528137 (CN); Hunan Brunp Recycling Technology Co., Ltd., Changsha, Hunan 410600 (CN); Hunan Brunp EV Recycling Co., Ltd., Changsha, Hunan 410600 (CN)
(72) Inventor: YU, Haijun, Foshan, Guangdong 528137 (CN); CHEN, Kang, Foshan, Guangdong 528137 (CN); LI, Aixia, Foshan, Guangdong 528137 (CN); XIE, Yinghao, Foshan, Guangdong 528137 (CN); ZHANG, Xuemei, Foshan, Guangdong 528137 (CN); LI, Changdong, Foshan, Guangdong 528137 (CN)
(74) Representative: Regimbeau
(86) International application number: PCT/CN2022/112244
(87) International publication number: WO 2023/071413

(57) **Abstract**

A carbon emission monitoring system. The system comprises an oxidation device (1), a gas mixing device (2), a gas measurement device (3), and a control device; the oxidation device (1), the gas mixing device (2), and the gas measurement device (3) are sequentially connected; the control device is separately connected to the oxidation device (1), the gas mixing device (2), and the gas measurement device (3); the input end of the oxidation device (1) receives waste gas, and the output end of the gas measurement device (3) discharges waste gas. The system can separately thoroughly oxidize and mix waste gas to be discharged, so that the waste gas can be oxidized more completely to reduce residual organic matter in the waste gas, and the system can perform secondary oxidation on the gas in sampling test, thereby improving the monitoring accuracy and monitoring efficiency.

## Description

### TECHNICAL FIELD

The invention relates to the technical field of recycling and treatment of waste battery materials, and in particular, to a carbon emission monitoring system.

### BACKGROUND

The recycling and processing industry of batteries belongs to the chemical engineering industry. Various organic solvents are utilized in the process, and it is prone to produce more carbon emissions during their treatment, posing a hazard to a certain extent. In order to reduce the hazard of the carbon emissions, it is necessary to conduct treatment on the waste gas (for example, adopting regenerative catalytic combustion technology) for environmental protection, before carbon is discharged into the atmosphere in the form of carbon dioxide through conduits.

During the discharge process, if the concentration of carbon dioxide is too high, it is very likely to cause environmental pollution and problems such as the greenhouse effect. To avoid problems such as environmental pollution, it is necessary to monitor real-time emissions continuously during the discharge process. At present, a commonly used monitoring method is to set a corresponding gas collector in a waste gas discharge pipe, collect a certain volume of the waste gas through the gas collector, monitor the carbon dioxide content in the collected waste gas, and finally determine an actual total carbon dioxide emission amount by calculating according to the proportion of the collected waste gas.

Currently, commonly used monitoring methods have the following technical problems: the gas is in a discrete state during the discharge process, and the process may be affected by many factors (size of the pipe, temperature, and pressure, etc.). It is difficult to accurately reflect the actual emission by quantitatively monitoring real-time carbon emissions at a single monitoring spot, which will lead to a too large error in the monitoring results to accurately determine real-time carbon emissions. Besides, in the treatment process of waste gas, there are still unoxidized organic matter remaining in the waste gas, and the organic matter can be re-oxidized in the air during the emission process to produce a certain amount of carbon dioxide, further increasing the error between monitored emissions and actual emissions.

### SUMMARY

The present invention provides a carbon emission monitoring system, which can completely catalyze and oxidize all organic matters in waste gas, and stir and measure a fully oxidized waste gas, so as to improve the monitoring accuracy.

A first aspect of the embodiments of the present invention provides a carbon emission monitoring system, including: an oxidation treatment device, a gas mixing device, a gas measurement device, and a control device.

The oxidation treatment device, the gas mixing device, and the gas measurement device are connected in sequence, and the control device is respectively connected with the oxidation treatment device, the gas mixing device, and the gas measurement device. An input end of the oxidation treatment device receives waste gas, and an output end of the gas measurement device discharges the waste gas.

In an implementation of the first aspect, the gas measurement device includes a gas buffer box, a catalytic filter box, a thermostat and a gas buffer tank. The gas buffer box, the catalytic filter box and the thermostat are arranged in sequence from bottom to top, and the gas buffer tank is communicated with the gas buffer box through a pipe.

In an implementation of the first aspect, the catalytic filter box is provided with a measurement chamber, a catalytic flow chamber and a sampling chamber.

The catalytic flow chamber is provided with a helical catalytic pipe, the top pipe opening of the catalytic pipe communicates with the top of the measurement chamber, and the bottom pipe opening of the catalytic pipe communicates with the top of the sampling chamber, such that the gas sampled from the sampling chamber enters from the bottom pipe opening of the catalytic pipe and a catalytic oxidation is performed on the gas during its rotating and rising process.

In an implementation of the first aspect, a sampling gas inlet opening is provided at the bottom of the sampling chamber, and at least one filter screen and at least one catalytic oxidation screen are sequentially provided on the top of the sampling gas inlet opening.

In an implementation of the first aspect, the measurement chamber is provided with a thermometer, a measurement bin and a gas outlet opening which are connected in sequence.

In an implementation of the first aspect, the gas mixing device includes a mixing box. A first mixing plate, a second mixing plate and a third mixing plate are arranged in the middle of the mixing box in sequence. The space between a mixing gas inlet opening of the mixing box and a side of the first mixing plate forms a stirring chamber, the space between the first mixing plate and the second mixing plate forms a first mixing chamber, the space between the second mixing plate and the third mixing plate forms a second mixing chamber, and the space between the third mixing plate and the mixing gas outlet opening of the mixing box forms a discharge chamber.

Here, each of two ends of the first mixing plate is provided with a first guide opening for discharging the mixing gas, a second guide opening is provided at the middle of the second mixing plate for discharging the mixing gas, and the third mixing plate is provided with serval third guide openings for discharging the mixing gas. The radius of the second guide opening is larger than the radius of the first guide opening, and the radius of the first guide opening is larger than the radius of the third guide opening.

In an implementation of the first aspect, the mixing gas inlet opening of the mixing box is provided with a gas inlet guide pipe, and the side of the first mixing plate facing the stirring chamber is provided with a reflex guide cylinder. The gas inlet guide pipe is arranged in the reflex guide cylinder.

In an implementation of the first aspect, the gas inlet guide pipe is conical, the inner wall of the gas inlet guide pipe is provided with an inner spiral plate, and the outer wall of the gas inlet guide pipe is provided with an outer spiral plate, the spiral direction of the inner spiral plate and the outer spiral plate are opposite.

In an implementation of the first aspect, the discharge chamber is provided with a conical net, and the cone head at the bottom of the conical net is connected to the third mixing plate, so as to collect the mixing gas discharged from the serval third guide opening.

In an implementation of the first aspect, a flow meter, a waste gas processor and a waste gas discharge pipe are further provided. An end of the gas buffer box is connected to the flow meter, the waste gas processor and the waste gas discharge pipe in sequence. The gas buffer box is provided with a thermometer.

Compared with the existing technology, the carbon emission monitoring system provided by the embodiment(s) of the present invention has the beneficial effects that the system can fully oxidize and mix the waste gas to be discharged, such that the waste gas can be oxidized more completely to reduce a residual organic matter in the gas. And a re-oxidation of the sampled gas is carried out during a sampling and detection process, which can improve monitoring accuracy and monitoring efficiency.

### BRIEF DESCRIPTION OF DRAWINGS

FIG. 1 is a schematic structural diagram of a carbon emission monitoring system provided by an embodiment of the present invention;
FIG. 2 is a schematic structural diagram of a gas measurement device provided by an embodiment of the present invention;
FIG. 3 is a schematic structural diagram of a catalytic filter box provided by an embodiment of the present invention;
FIG. 4 is a schematic structural diagram of a mixing box provided by an embodiment of the present invention; and
FIG. 5 is a schematic structural diagram of a control device according to an embodiment of the present invention;

In the figure: oxidation treatment device 1, gas mixing device 2, gas measurement device 3, flow meter 4, waste gas processor 5, waste gas discharge pipe 6, mixing box 21, first mixing plate 22, second mixing plate 23, third mixing plate 24, first guide opening 25, second guide opening 26, third guide opening 27, gas inlet guide pipe 28, reflux guide cylinder 29, conical net 210, inner spiral plate 281, outer spiral plate 282, gas buffer box 31, catalytic filter box 32, thermostat 33, gas buffer tank 34, catalytic pipe 321, thermometer 322, measurement bin 323, gas outlet opening 324, sampling gas inlet opening 325, suction pump 326, filter screen 327, catalytic oxidation screen 328.

### DETAILED DESCRIPTION

The technical schemes in the embodiments of the present invention will be clearly and completely described below with reference to the accompanying drawings in the embodiments of the present invention. Obviously, the described embodiments are only a part of the embodiments of the present invention, but not all the embodiments. Based on the embodiments of the present invention, all other embodiments obtained by a person having ordinary skills in the art without creative efforts shall fall within the protection scope of the present invention.

The currently commonly used monitoring methods have the following technical problems: since gas is in a discrete state during a discharge process, and gas discharge is affected by many factors (the size of a pipe, temperature, pressure, etc.), it is difficult to quantitatively monitor emissions under an actual situation by adopting a single monitoring point, which results in a large error in the monitoring results and fails to accurately determine real-time carbon emissions. And in a waste gas treatment process, there are still unoxidized organic matter remaining in the waste gas, and the organic matter can be re-oxidized in the air during an emission process to produce a certain amount of carbon dioxide, further enlarging the error between the monitored emissions and actual emissions.

In order to solve the above problems, a carbon emission monitoring system provided by the embodiments of the present application will be introduced and described in detail below through the following specific embodiments.

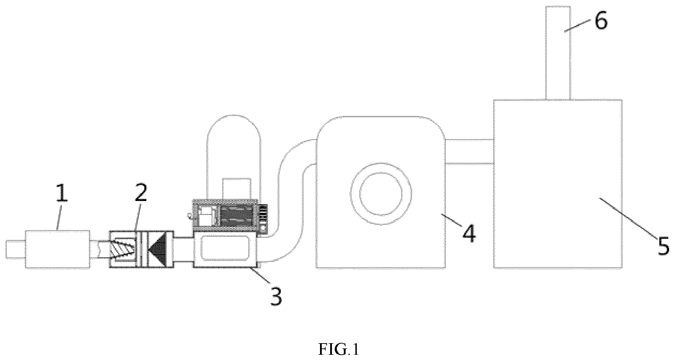

A schematic structural diagram of a carbon emission monitoring system referring to FIG. 1 provided by an embodiment of the present invention is illustrated.

In an embodiment, the carbon emission monitoring system can include: an oxidation treatment device 1, a gas mixing device 2, a gas measurement device 3 and a control device.

The oxidation treatment device 1, the gas mixing device 2, and the gas measurement device 3 are connected in sequence, and the control device is respectively connected with the oxidation treatment device 1, the gas mixing device 2, and the gas measurement device 3. An input end of the oxidation treatment device 1 receives waste gas, and an output end of the gas measurement device 3 discharges the waste gas.

When using the system, waste gas enters the oxidation treatment device 1 to be completely oxidized by the oxidation treatment device 1, and then the resulting oxidation-treated waste gas enters the gas mixing device, where the oxidation-treated waste gas is stirred and mixed to avoid an incomplete oxidation before the oxidation-treated waste gas enters the gas measurement device 3 for carbon emission measurement. The complete oxidation treatment reduces residual organic matter in the waste gas, and the probability of a residual organic matter being oxidized in a subsequent discharge process can also be reduced. At the same time, the oxidation-treated waste gas is stirred to further mix the waste gas with the organic matter, so as to further oxidize the untreated organic matter, and further reduce the probability of a residual organic matter being oxidized in a subsequent discharge process, thereby improving the accuracy of a subsequent detection. The entire process is controlled by the control device, which can facilitate user operation and improve processing efficiency.

It should be noted that the control device may be arranged outside the system or inside the system, and if the control device is arranged inside the system, it may be arranged in the gas measurement device 3. Since the control device can be set in various ways, it is not marked in the drawings, and the setting ways can be adjusted according to an actual need of users.

In this embodiment, the oxidation treatment device 1 may include a regenerative catalytic combustion unit as an oxidation unit for completely oxidizing the waste gas.

In an embodiment referring to FIG. 1, the carbon emission monitoring system may further include a flow meter 4, a waste gas processor 5 and a waste gas discharge pipe 6, and the gas measurement device 3 is connected to the flow meter, the waste gas processor and the waste gas discharge pipe in sequence.

When the oxidation-treated waste gas enters the gas measurement device 3, part of the gas is sampled and detected by the gas measurement device 3, and the remaining waste gas sequentially enters the flow meter, the waste gas processor and the waste gas discharge pipe. The flow meter can calculate the capacity of the remaining waste gas which is discharged; the waste gas processor can further treat the remaining waste gas to reduce its pollution to the environment before the gas is finally discharge into the atmosphere through the waste gas discharge pipe.

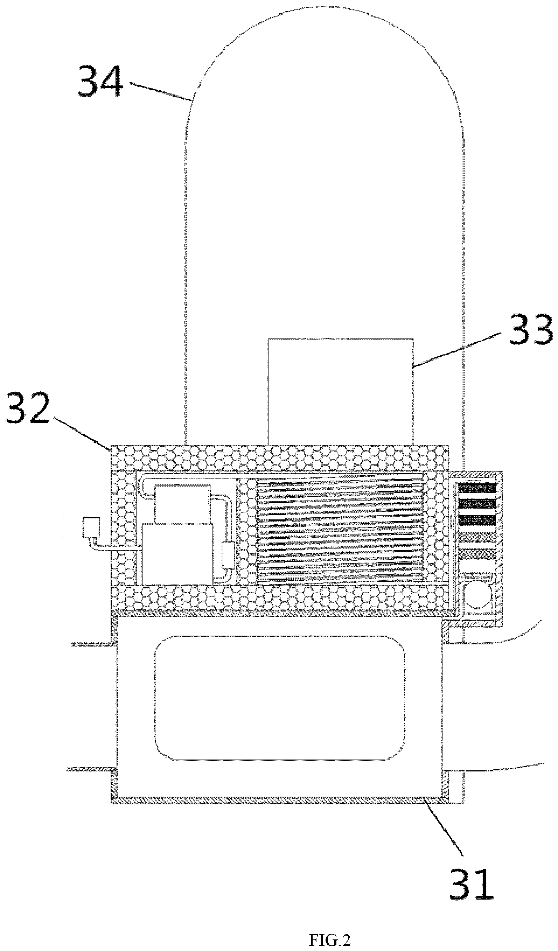

The waste gas is sampled for detection during the monitoring. Air pressures of the waste gas is unstable when the gas is discharged. In order to stabilize the air pressure during the sampling to improve the sampling consistency, a gas measurement device referring to FIG. 2 is provided by an embodiment of the present invention. A schematic structural diagram of the gas measurement device according to an embodiment of the present invention is illustrated in FIG. 2. In an embodiment, the gas measurement device 3 includes a gas buffer box 31, a catalytic filter box 32, a thermostat 33 and a gas buffer tank 34. The gas buffer box 31, the catalytic filter box 32 and the thermostat 33 are connected in sequence from bottom to top, and the gas buffer tank 34 is communicated with the gas buffer box 31 through a connecting pipe 35.

Specifically, the gas buffer box 31 can be configured to buffer the air pressure of the waste gas; the catalytic filter box 32 can be configured to detect the carbon emission capacity of the gas; the thermostat 33 is configured to keep the gas buffer box 31 and the catalytic filter box 32 at a stable operating temperature. The gas buffer tank 34 is configured for buffering the air pressure in the gas buffer box 31.

The gas buffer box 31 can buffer the air pressure between the catalytic filter box 32 and the gas buffer box 31 when the gas is discharge, so as to maintain the air pressure stability in the catalytic filter box 32 and improve the monitoring efficiency.

Referring to FIG. 2, an end of the gas buffer box 31 may be connected to the flow meter, the waste gas processor and the waste gas discharge pipe in sequence. The gas passes through the gas buffer box 31, the flow meter, the waste gas processor and the waste gas discharge pipe in sequence, and finally is discharged.

When using the system, in order to determine the temperature of the waste gas, in an embodiment, the gas buffer box 31 is provided with a thermometer.

The gas has different volume at different temperature, and the gas buffer box 31 has a larger cross section size than the pipe, which is approximately the same as the gas buffer tank 34. During the monitoring process, the air pressure in the gas buffer box 31 may vary in a large range, resulting in inaccurate sampling. The thermostat is set to reduce the measurement fluctuation caused by temperature. The thermostat 33 can ensure each device at an identical temperature when monitoring is carried out, such that the measurement by the catalytic filter box 32 is more accurate.

In an embodiment, the thermostat 33 may be a water medium constant temperature unit, and the temperature of the water medium in the thermostat 33 is set within a range of ±5 degrees from the ambient temperature.

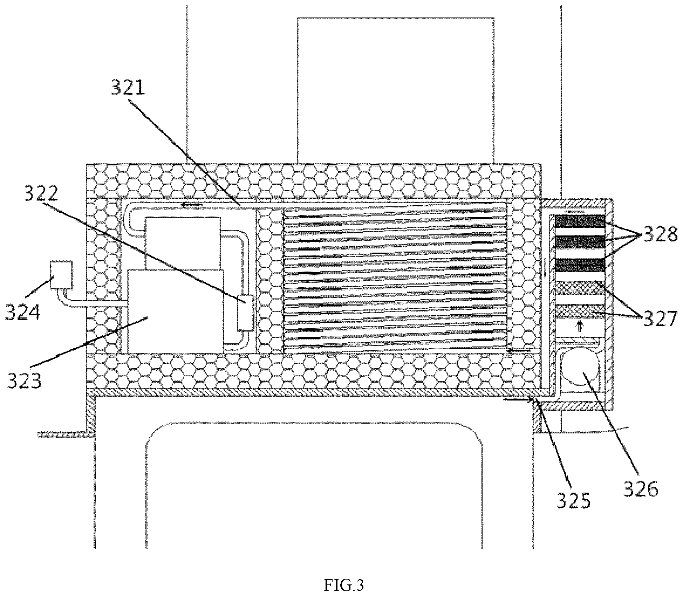

Referring to FIG. 3, a schematic structural diagram of a catalytic filter box provided by an embodiment of the present invention is shown, and the catalytic filter box 32 is provided with a measurement chamber, a catalytic flow chamber and a sampling chamber.

The catalytic flow chamber is provided with a helical catalytic pipe 321, the top conduit opening of the catalytic pipe 321 communicates with the top of the measurement chamber, and the bottom conduit opening of the catalytic pipe 321 communicates with the top of the sampling chamber. The gas sampled from the sampling chamber enters from the bottom pipe opening of the catalytic pipe 321 and a catalytic oxidation is performed on the gas rotating and rising in the conduit.

When the system is applied, the sampling chamber can extract an appropriate amount of gas from the gas buffer box 31, and then deliver the gas to the catalytic flow chamber, which is then transported to the measurement chamber for measurement. Since the catalytic flow chamber is provided with a helical catalytic pipe 321, the gas rotates and ascends in the catalytic pipe 321 for transmission, and can be further oxidized during the transmission process to further reduce the residual organic matter in the gas to improve the monitoring accuracy.

Referring to FIG. 3, the measurement chamber is provided with a thermometer 322, a measurement bin 323 and a gas outlet opening 324 which are connected in sequence. Specifically, the thermometer 322 can be configured to detect the temperature of the sampled waste gas, the measurement bin 323 can be configured to detect the carbon emission content of the sampled waste gas, and the gas outlet opening 324 can be configured to discharge the sampled waste gas.

In an embodiment, an air stone may be provided at a gas nozzle of the gas outlet opening 324.

In an implementation, the measurement bin 323 may be provided with a wet flow meter and a carbon dioxide meter configured for detecting the humidity and carbon dioxide content of the gas, respectively.

Referring to FIG. 3, in an embodiment, a sampling gas inlet opening 325 is provided at the bottom of the sampling chamber, and a suction pump 326 may be provided at the rear end of the sampling gas inlet opening 325. At least one filter screen 327 and at least one catalytic oxidation screen 328 are arranged in sequence on the top of the outlet of the suction pump 326.

Referring to FIG. 3, in an embodiment, two filter screens 327 and three catalytic oxidation screens 328 are provided.

In yet another embodiment, the wet flow meter in the catalytic filter box 32 may be set at the inlet of the sampling gas inlet opening 325 to monitor the humidity of the input gas.

When the system is applied, the water medium in the thermostat 33 maintains a constant temperature and communicates with an air input pipe. The suction pump 326 communicates with the gas buffer box 31 through the sampling gas inlet opening 325 to extract gas with low variable pressure from the gas buffer box 31. The sampled gas passes through the wet flow meter, the filter screen 327 and the catalytic oxidation screen 328 for further catalytic oxidation, and then is detected by the wet flow meter and the carbon dioxide meter. The gas after being detected is discharged through the air outlet opening 324 into the atmosphere. The operating temperature can be kept stable by the thermostat 33 during the whole process.

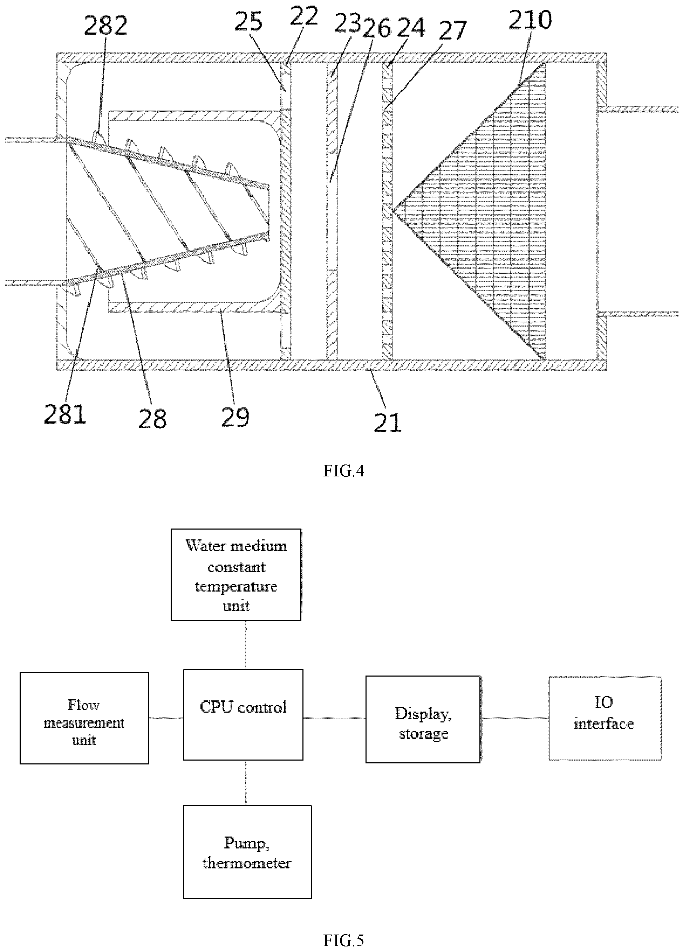

Referring to FIG. 4, a schematic structural diagram of a mixing box provided by an embodiment of the present invention is shown. The gas mixing device 2 includes a mixing box 21. A first mixing plate 22, a second mixing plate 23 and a third mixing plate 24 are provided in the mixing box 21. The space between the mixing gas inlet opening of the mixing box 21 and a side of the first mixing plate 22 forms a stirring chamber. The space between the first mixing plate 22 and the second mixing plate 23 forms a first mixing chamber. The space between the second mixing plate 23 and the third mixing plate 24 forms a second mixing chamber. The space between the third mixing plate 24 and the mixing gas outlet opening of the mixing box 21 forms a discharge chamber.

In an embodiment, each of two ends of the first mixing plate 22 is provided with a first guide opening 25 for discharging the mixing gas, a second guide opening 26 is provided in the middle of the second mixing plate 23 for discharging the mixed gas, and the third mixing plate 24 is provided with serval third guide openings 27 for discharging the mixed gas.

Here, the stirring chamber can be configured to stir the gas, the first mixing chamber and the second mixing chamber can be configured to mix the stirred gas, and the discharge chamber can be configured to discharge the mixed gas to the gas measurement device 3.

When the system is applied, the gas treated by the oxidation treatment device 1 is output to the mixing box 21, and passes through the stirring chamber, the first mixing chamber, the second mixing chamber and the discharge chamber, respectively, for treatment of stirring, mixing and discharging, and finally is measured.

In an embodiment, in order to improve the gas mixing efficiency, the flow rate of the gas can be adjusted. Referring to FIG. 4, in an implementation, the radius of the second guide opening 26 is greater than the radius of the first guide opening 25, and the radius of the first guide opening 25 is greater than that of the third guide opening 27.

Since the radii of the first guide opening 25, the second guide opening 26 and the third guide opening 27 are different, the speeds of the gas passing through the first guide opening 25, the second guide opening 26 and the third guide opening 27 are different, such that the gas passes through the first guide opening 25, the second guide opening 26 and the third guide opening 27 with different velocities. The velocity difference is generated to ensure a better mixing effect of the gas and improve the mixing efficiency of the gas.

In an embodiment, in order to increase the gas flow path to improve the mixing effect of the gas, referring to FIG. 4, the mixing gas inlet opening of the mixing box 21 is provided with a gas inlet guide pipe 28, the side of the first mixing plate 22 facing the stirring chamber is provided with a reflux guide cylinder 29, and the gas inlet guide pipe 28 is arranged in the reflux guide cylinder 29.

Specifically, the oxidization-treated gas enters the gas inlet guide pipe 28, flows from the bottom of the reflux guide cylinder 29 to its lateral sides followed by passing through the end opening of the reflux guide cylinder 29. The gas then passes through the outer wall of the reflux guide cylinder 29 and enters the first guide opening 25.

In order to further improve the mixing effect of the gas, referring to FIG. 4, in an embodiment, the gas inlet guide pipe 28 is conical, and the inner wall of the gas inlet guide pipe 28 is provided with an inner spiral plate 281. The outer wall of the gas inlet guide pipe 28 is provided with an outer spiral plate 282, and spiral directions of the inner spiral plate 281 and the outer spiral plate 282 are opposite.

Specifically, the inner spiral plate 281 and the outer spiral plate 282 can be configured to respectively drive the gas to rotate and flow, so as to achieve a mixing effect.

When the mixing gas enters the discharge chamber through the multiple third guide openings 27, in order to collect the gas, in an embodiment referring to FIG. 4, the discharge chamber is provided with a conical net 210, and the cone head at the bottom of the conical net 210 is connected to the third mixing plate 24 to collect the mixed gas discharged from the plurality of the third guide openings 27.

Referring to FIG. 5, a schematic structural diagram of a control device provided by an embodiment of the present invention is shown. In an embodiment, the control device may include a CPU control chip and a display that are connected to each other, where the display can connect with an external IO interface, and the CPU control chip can be respectively connected with the oxidation treatment device 1, the gas mixing device 2, and the gas measurement device 3. In an implementation, the CPU control chip can connect with the flow meters and thermometers, the thermostat 33, and the suction pump.

An embodiment of the present invention provides a carbon emission monitoring system, and the beneficial effect is that the present invention can completely oxidize and mix the waste gas to be discharged, such that the waste gas can be oxidized more completely, so as to reduce residual organic matter in the waste gas, and a re-oxidation of the gas in the sampling and detection step is adopted to improve the monitoring accuracy and efficiency.

The above described are some embodiments of the present invention. It should be pointed out that for a person having ordinary skills in the art, without departing from the principles of the present invention, several improvements and modifications can be made, and these improvements and modifications may also be regarded as they fall in the protection scope of the present invention.

## Claims

1. A carbon emission monitoring system, comprising:
an oxidation treatment device, a gas mixing device, a gas measurement device and a control device,
wherein, the oxidation treatment device, the gas mixing device, and the gas measurement device are connected in sequence, and the control device is respectively connected with the oxidation treatment device, the gas mixing device, and the gas measurement device; an input end of the oxidation treatment device receives waste gas, and an output end of the gas measurement device discharges the waste gas.

2. The carbon emission monitoring system according to claim 1, wherein the gas measurement device comprises a gas buffer box, a catalytic filter box, a thermostat and a gas buffer tank, the gas buffer box, the catalytic filter box and the thermostat are connected in sequence from bottom to top, and the gas buffer tank is communicated with the gas buffer box through a connecting pipe.

3. The carbon emission monitoring system according to claim 2, wherein the catalytic filter box is provided with a measurement chamber, a catalytic flow chamber and a sampling chamber,
wherein the catalytic flow chamber is provided with a helical catalytic pipe, a top pipe opening of the catalytic pipe communicates with a top of the measurement chamber, and a bottom pipe opening of the catalytic pipe communicates with a top of the sampling chamber, such that gas sampled from the sampling chamber enters from the bottom pipe opening of the catalytic pipe and a catalytic oxidation is performed on the gas during a rotating and rising process of the gas.

4. The carbon emission monitoring system according to claim 3, wherein a sampling gas inlet opening is provided at a bottom of the sampling chamber, and at least one filter screen and at least one catalytic oxidation screen are sequentially provided on the top of the sampling gas inlet opening.

5. The carbon emission monitoring system according to claim 3, wherein the measurement chamber is provided with a thermometer, a measurement bin and a gas outlet opening which are connected in sequence.

6. The carbon emission monitoring system according to claim 1, wherein the gas mixing device comprises a mixing box; a first mixing plate, a second mixing plate and a third mixing plate are arranged in middle of the mixing box in sequence; a space between a mixing gas inlet opening of the mixing box and a side of the first mixing plate forms a stirring chamber, a space between the first mixing plate and the second mixing plate forms a first mixing chamber, a space between the second mixing plate and the third mixing plate forms a second mixing chamber, and a space between the third mixing plate and a mixing gas outlet opening of the mixing box forms a discharge chamber,
wherein, each of two ends of the first mixing plate is provided with a first guide opening for discharging mixing gas, a second guide opening is provided at a middle of the second mixing plate for discharging the mixing gas, and the third mixing plate is provided with serval third guide openings for discharging the mixing gas; and a radius of the second guide opening is larger than a radius of the first guide opening, and the radius of the first guide opening is larger than a radius of the third guide opening.

7. The carbon emission monitoring system according to claim 6, wherein the mixing gas inlet opening of the mixing box is provided with a gas inlet guide pipe, the side of the first mixing plate facing the stirring chamber is provided with a reflux guide cylinder, and the gas inlet guide pipe is arranged in the reflux guide cylinder.

8. The carbon emission monitoring system according to claim 7, wherein the gas inlet guide pipe is conical, and an inner wall of the gas inlet guide pipe is provided with an inner spiral plate, an outer wall of the gas inlet guide pipe is provided with an outer spiral plate, and spiral directions of the inner spiral plate and the outer spiral plate are opposite.

9. The carbon emission monitoring system according to claim 6, wherein the discharge chamber is provided with a conical net, and a cone head at a bottom of the conical net is connected to the third mixing plate, so as to collect the mixing gas discharged from the serval third guide opening.

10. The carbon emission monitoring system according to any one of claims 2 to 5, further comprising a flow meter, a waste gas processor and a waste gas discharge pipe, wherein an end of the gas buffer box is connected to the flow meter, the waste gas processor and the waste gas discharge pipe in sequence and the gas buffer box is provided with a thermometer.
